# EUROPÄISCHE PATENTSCHRIFT

(11) **EP 2 414 126 B1**
(45) Veröffentlichungstag und Bekanntmachung des Hinweises auf die Patenterteilung: **10.05.2017**
(21) Anmeldenummer: 10722285.3
(22) Anmeldetag: 26.03.2010
(51) Int. Cl.: B23K 9/02, B23K 9/095, B23K 9/127, B23K 9/32, A61F 9/06, G06F 3/048, G06F 3/01, G06F 3/033, B23K 9/10, G06K 9/00

(54) **VERFAHREN UND VORRICHTUNG ZUR FERNBEDIENUNG EINER MIT EINEM HANDBETÄTIGTEN ARBEITSGERÄT VERBUNDENEN STROMQUELLE**
METHOD AND APPARATUS FOR THE REMOTE CONTROL OF A POWER SOURCE CONNECTED TO A MANUALLY ACTUATED IMPLEMENT
PROCÉDÉ ET DISPOSITIF PERMETTANT L'UTILISATION D'UNE SOURCE DE COURANT RELIÉE À UN OUTIL À MAIN

(30) Priorität: 31.03.2009 AT 5092009
(43) Veröffentlichungstag der Anmeldung: 08.02.2012
(73) Patentinhaber: Fronius International GmbH, 4643 Pettenbach (AT)
(72) Erfinder: SCHIEFERMÜLLER, Robert, A-4611 Buchkirchen (AT); BRUNMAYR, Wolfgang, A-4600 Wels (AT); POINTNER, Christian, A-4720 Kallham (AT)
(74) Vertreter: Sonn & Partner Patentanwälte
(86) Internationale Anmeldenummer: PCT/AT2010/000090
(87) Internationale Veröffentlichungsnummer: WO 2010/111722

(56) Entgegenhaltungen:
- EP-A1- 2 022 592
- WO-A1-2007/009131
- WO-A1-2007/137310
- WO-A2-03/104965
- DE-A1-102006 048 166
- JP-A- 2004 160 578
- US-A- 2 526 597
- US-A- 4 266 114
- US-A- 4 380 696
- US-A1- 2007 070 358
- US-A1- 2008 082 179
- US-B1- 6 506 050
- US-B1- 6 734 393

## Beschreibung

Die Erfindung betrifft ein Verfahren zur Bedienung einer mit einem handbetätigten Schweißbrenner verbundenen Stromquelle durch einen Benutzer nach den Oberbegriffen der Ansprüche 1, und 12 und eine Kombination eines handbetätigten Schweißbrenners und einer Stromquelle nach dem Oberbegriff des Anspruches 13. Die Bedien- und Anzeigeeinheit bzw. das Benutzer- oder Userinterface ist vorzugsweise durch einen Touchscreen, der die Anzeige- und Bedienfunktion gleichzeitig erfüllt, gebildet.

Die WO 2007/009131 A1, Basis für den Oberbegriff der Ansprüche 1, 12 und 13, beschreibt ein Verfahren und eine Vorrichtung zur Bestimmung der Position des Schweißbrenners im dreidimensionalen Raum, vorzugsweise zur Qualitätskontrolle. Dabei besteht die Möglichkeit, automatisch in Abhängigkeit der Position des Schweißbrenners bzw. der Position der Schweißnaht, Schweißparameter an der Sprungquelle einzustellen bzw. zu verändern, wodurch Fehleinstellungen vom Schweißer ausgeschlossen werden können.

Allgemein ist es aus dem Stand der Technik bekannt, eine Stromquelle über Bedienelemente, welche an einem Benutzerinterface mit den Fingern des Benutzers betätigt werden, zu bedienen.

Die WO 2007/009131 A1 beschreibt ein Schweißverfahren bei dem die Position des Schweißbrenners während des Schweißens bestimmt wird. Eine Steuerung über die Bewegung des Schweißbrenners ist nicht geoffenbart.

Die US 6 506 050 B1 beschreibt ein zahnärztliches Behandlungsinstrument das auch als Eingabegerät zur Betätigung des Steuergeräts herangezogen werden kann.

Andere Einrichtungen, bei welchen die Position eines Eingabegeräts erfasst und auf eine Anzeigevorrichtung übertragen wird, sind beispielsweise aus der US 2007/0070358 A1 oder der WO 03/104965A2 bekannt geworden.

Die US 6,506,050 B1 betrifft ein dental Instrument, dessen Position automatisch erfasst wird und mit der ein Mauszeiger am Bildschirm bewegt werden kann. Ähnliche Navigationsvorrichtungen auf anderen technischen Gebieten sind beispielsweise aus der US 2007/0070358 A1 und der WO 03/104965 A2 bekannt geworden.

Die JP 2004/160578 A und US 4,380,696 A beschreiben eine Bedienfunktion an der Stromquelle eines Schweißgeräts, welche vor dem Schweißprozess durchgeführt wird.

Aus der US 6,734,393 B1 ist ein Schweißhelm mit einem Bildschirm zur Wiedergabe von Daten bekannt geworden.

Die Aufgabe der vorliegenden Erfindung liegt darin, eine schnelle und einfache Bedienung einer Stromquelle zu ermöglichen, ohne dabei eine allfällige Schutzbekleidung, insbesondere Schutzhandschuhe, entfernen zu müssen.

Die Aufgabe der Erfindung wird in verfahrensmäßiger Hinsicht dadurch gelöst, dass eine Bedienfunktion an der Stromquelle vor, nach oder zwischen mit dem Schweißbrenner durchgeführten Arbeiten aktiviert wird und bestimmte Funktionen der Stromquelle aus Sicherheitsgründen deaktiviert werden, wonach die Bewegung des Schweißbrenners im Raum erfasst, die aus der Bewegung des Schweißbrenners resultierenden Daten in der Stromquelle ausgewertet werden, und eine aus der Bewegung resultierende Position des Schweißbrenners einem Bedienelement der Bedien- und Anzeigeeinheit zugeordnet wird, die Zuordnung der Position zu dem Bedienelement an der Bedien- und Anzeigeeinheit gekennzeichnet wird, und das zugeordnete Bedienelement bestätigt wird, nach der Bestätigung des Bedienelements der zumindest eine dem Bedienelement zugeordnete Parameter der Stromquelle eingestellt bzw. verändert wird, und danach die Bedienfunktion an der Stromquelle wieder deaktiviert wird, und der Schweißbrenner wieder zweckmäßig benutzt wird. Es muss also die Bedienfunktion an der Stromquelle erst aktiviert werden, worauf eine Bedienung über die Bewegung des Schweißbrenners erfolgen kann. Vorteilhaft ist hierbei, dass eine äußerst einfache Bedienung der Stromquelle auch mit einer allfälligen Schutzausrüstung bzw. Schutzbekleidung des Benutzers berührungslos mit dem Schweißbrenner durchgeführt werden kann. Nach der Zuordnung eines Bedienelements der Bedien- und Anzeigeeinheit durch entsprechende Bewegung des Schweißbrenners wird das zugeordnete Bedienelement durch den Benutzer bestätigt. Eine allenfalls vorhandene Schutzabdeckung der Bedien- und Anzeigeeinheit an der Stromquelle muss für eine Bedienung ebenfalls nicht entfernt werden. Schließlich ist eine derartige Bedienung auch unabhängig von der Ausführung der Bedien- und Anzeigeeinheit (Touchscreen, Taster, Drehknöpfe, ...). Weiters kann der Benutzer die Position bestimmen, von welcher die Bedienung erfolgt. Dadurch muss der Benutzer beispielsweise eine bestimmte Arbeitsposition nicht verlassen, um eine Bedienung durchzuführen.

Die Aktivierung der Bedienfunktion an der Stromquelle kann durch die Kennzeichnung eines zur Lage des Schweißbrenners korrespondierenden Referenzpunkts, die Betätigung eines Betätigungselements am Schweißbrenner, die Positionierung des Schweißbrenners in einem definierten Abstand zur Stromquelle oder die Durchführung einer kurzen, ruckartigen Bewegung mit dem Schweißbrenner erfolgen.

Vorteilhafterweise werden die Bewegungen des Schweißbrenners im Raum dadurch erfasst, dass die Bewegungen zumindest in zwei Dimensionen des Raums oder der Winkel des Schweißbrenners im Raum ausgewertet wird.

Für eine Rückmeldung an den Benutzer wird bei Zuordnung der Position zu einem Bedienelement dieses vorzugsweise an der Bedien- und Anzeigeeinheit gekennzeichnet bzw. markiert.

Die Bestätigung des zugeordneten Bedienelements kann durch die Betätigung eines Betätigungselements am Schweißbrenner, durch eine Bewegung des Schweißbrenners, oder durch Berührung des Bedienelements an der Bedien- und Anzeigeeinheit durch den Schweißbrenner erfolgen.

Gemäß einem weiteren Merkmal der Erfindung werden die aus der Bewegung des Schweißbrenners resultierenden Daten in der Stromquelle ausgewertet.

Dabei können die Daten über einen in einem Schlauchpaket zwischen dem Schweißbrenner und der Stromquelle angeordneten Bus zur Stromquelle übertragen werden.

Gemäß einem weiteren Merkmal der Erfindung wird mit der Bedienfunktion ein mit der Stromquelle verbundenes Head-up-Display aktiviert und zumindest ein Teil der Bedien- und Anzeigeeinheit im Head-up-Display angezeigt und mit Deaktivierung der Bedienfunktion das Head-up-Display wieder deaktiviert.

Gelöst wird die erfindungsgemäße Aufgabe in verfahrensmäßiger Hinsicht auch dadurch, dass eine Bedienfunktion an der Stromquelle vor, nach oder zwischen mit dem Schweißbrenner durchgeführten Arbeiten aktiviert wird, und bestimmte Funktionen der Stromquelle aus Sicherheitsgründen deaktiviert werden, wonach die Bewegung des Schweißbrenners im Raum erfasst und die erfassten Bewegungen des Schweißbrenners zur Erkennung eines Bewegungsablaufs aufgenommen und mit in der Stromquelle hinterlegten Bewegungsabläufen verglichen werden, und bei Übereinstimmung ein dem hinterlegten Bewegungsablauf zugeordneter Funktionsablauf ausgeführt wird, und danach die Bedienfunktion an der Stromquelle wieder deaktiviert wird, und der Schweißbrenner wieder zweckmäßig benutzt wird.

Schließlich wird die Aufgabe auch durch eine oben genannte Stromquelle gelöst, wobei die Auswerteeinheit zur Durchführung des oben beschriebenen Verfahrens ausgebildet ist. Vorteilhaft ist hierbei, dass die Bedienung vorzugsweise ausschließlich über die Bewegungen des handbetätigten Schweißbrenners erfolgt, ohne dass beispielsweise die Betätigung von Tastern erforderlich ist. Weiter sich daraus ergebende Vorteile können aus der obigen Beschreibung und den nachfolgenden Beispielen entnommen werden.Die vorliegende Erfindung wird anhand der beigefügten, schematischen Zeichnungen näher erläutert.

Darin zeigen:
Fig. 1 eine schematische Darstellung einer Frontansicht einer Stromquelle bzw. eines Schweißgeräts;
Fig. 2 eine schematische Darstellung einer Stromquelle bzw. eines Schweißgeräts mit einem Schweißbrenner in einem Raum;
Fig. 3 bis 5 verschiedene Ansichten einer Bedien- und Anzeigeeinrichtung einer Stromquelle;
Fig. 6 bis 8 weitere Ansichten einer Bedien- und Anzeigeeinrichtung einer Stromquelle gemäß der vorliegenden Erfindung; und
Fig. 9 eine Ansicht eines Schweißhelms zur Ausführung eines Bedienverfahrens.

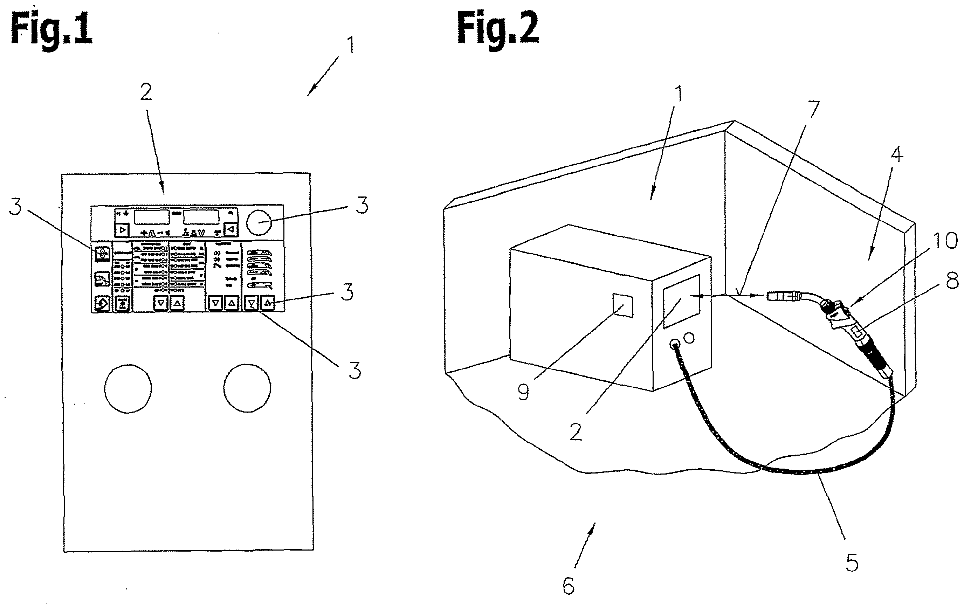

Einführend wird festgehalten, dass gleiche Teile des Ausführungsbeispiels mit gleichen Bezugszeichen versehen werden, wobei die in der gesamten Beschreibung enthaltenen Offenbarungen sinngemäß auf gleiche Teile mit gleichen Bezugszeichen übertragen werden können. Weiters können auch Einzelmerkmale aus dem gezeigten Ausführungsbeispiel bzw. aus den gezeigten Ausführungsbeispielen für sich eigenständige, erfindungsgemäße Lösungen darstellen. Die Bedienung einer Stromquelle 1 erfolgt im Allgemeinen durch einen Benutzer über ein Benutzerinterface bzw. eine Bedien- und Anzeigeeinheit 2, indem der Benutzer die in der Bedien- und Anzeigeeinheit 2 integrierten Bedienelemente 3 betätigt. Zur Vornahme bestimmter Einstellungen an der Stromquelle 1, insbesondere vor einem durchzuführenden Schweißprozess, werden dabei die Bedienelemente 3 mit den Fingern des Benutzers betätigt. Dazu muss in den meisten Fällen der handbetätigte Schweißbrenner 4, und allenfalls auch ein Schutzhandschuh weggelegt werden, bevor die Einstellungen vorgenommen werden können. Häufig ist eine Vielzahl von Bedienelementen 3 an der Bedien- und Anzeigeeinheit 2 angeordnet, mit welchen eine große Anzahl an Parametern eingestellt werden können. Dementsprechend können die Bedienelemente 3 mit einem Schutzhandschuh meist nicht betätigt werden. Fig. 1 zeigt eine Frontansicht einer Stromquelle 1 mit einer Bedien- und Anzeigeeinheit 2 mit einigen beispielhaft bezeichneten Bedienelementen 3. Die Bedien- und Anzeigeeinheit 2 ist vorzugsweise durch einen sogenannten Touchscreen gebildet, welcher sowohl die Bedien- als auch Anzeigefunkion übernimmt. Unterhalb der Bedien- und Anzeigeeinheit 2 sind zwei Anschlüsse an der Stromquelle 1 dargestellt, an welche der handbetätigte Schweißbrenner 4 über ein Schlauchpaket 5 angeschlossen wird (siehe Figur 2).

Erfindungsgemäß wird die Bedienung durch Bewegungen des Schweißbrenners 4 in einer Hand des Benutzers im Raum 6 durchgeführt, indem nach Aktivierung einer Bedienfunktion an der Stromquelle 1 eine aus den Bewegungen resultierende Position automatisch der Bedien- und Anzeigeeinheit 2, insbesondere einem Bedienelement 3, zugeordnet wird.

Somit kann ein Parameter der Stromquelle 1 vom Benutzer direkt über den Schweißbrenner 4 eingestellt werden, als ob die Einstellung direkt mit den Bedienelementen 3 an der Bedien- und Anzeigeeinheit 2 vorgenommen würde. Beispielsweise führt ein Schweißer die Bewegungen mit einem Schweißbrenner oder einer anderen handbetätigten Komponente eines Schweißgeräts, wie zum Beispiel einem Fernregler, durch. Die Einstellungen über die erfindungsgemäße Bedienung können vor, nach oder zwischen den mit dem Schweißbrenner 4 durchgeführten Arbeiten vorgenommen werden, also beispielsweise vor, nach oder zwischen den Schweißprozessen. Eine Bedienung während des Schweißprozesses soll nicht durchgeführt werden können. Beispielsweise wird mit der Zündung eines Lichtbogens bzw. mit dem Anlegen einer Hochfrequenz zum Zünden des Lichtbogens die Bedienfunktion deaktiviert.

Der erfindungsgemäße Ablauf zur Bedienung der Stromquelle 1 durch Bewegungen eines Schweißbrenners 4 in einer Hand eines Benutzers im Raum 6 wird anhand der Fig. 2 bis 8 beschrieben. Der Benutzer hält den Schweißbrenner 4 in der Hand und kann mit den Bewegungen des Schweißbrenners 4 in einem sogenannten Bedienbereich die Bedien- und Anzeigeeinheit 2 der Stromquelle 1 bedienen. Der Bedienbereich kann dabei unterschiedlich definiert werden, wobei für den Schweißbrenner 4 im Bedienbereich immer ein Referenzpunkt definiert wird. Beispielsweise kann der Schweißbrenner 4 in einem definierten Abstand 7 zur Bedien- und Anzeigeeinheit 2 positioniert werden, wodurch ein Referenzpunkt definiert wird und automatisch auf die Bedienfunktion der Stromquelle 1 umgeschaltet bzw. diese aktiviert wird. Dadurch kann der Benutzer auf die Bedien- und Anzeigeeinheit 2 blicken und die Einstellungen kontrolliert vornehmen. Zum anderen kann auch über ein Betätigungselement 10 am Schweißbrenner 4 auf die Bedienfunktion umgeschaltet werden und gleichzeitig mit der Betätigung des Betätigungselements 10 ein Referenzpunkt definiert werden. Dabei kann ein eigenes Betätigungselement 10 am Schweißbrenner 4 angeordnet sein oder es können vorhandene Betätigungselemente 10 - beispielsweise zur Durchführung eines Schweißprozesses - herangezogen werden. Beispielsweise müssen vorhandene Betätigungselemente 10 für eine definierte Zeitdauer betätigt werden, wobei die Position des Schweißbrenners 4 von der Entfernung oder Richtung zur Bedien- und Anzeigeeinheit 2 vorzugsweise unabhängig ist. Die Umschaltung auf die Bedienfunktion kann aber auch durch entsprechende Ausrichtung des Schweißbrenners 4 in Richtung der Stromquelle 1 erfolgen. Dabei wird der Referenzpunkt definiert, sobald ein "Sichtkontakt" zwischen dem Schweißbrenner 4 und der Bedien- und Anzeigeeinheit 2 bzw. der Stromquelle 1 besteht. Eine Umschaltung ist auch durch eine kurze, ruckartige Bewegung mit dem Schweißbrenner 4, wie einem Klopfen, möglich. Durch das Umschalten wird somit bewirkt, dass der Schweißbrenner 4 beispielsweise von einem Schweißmodus in einen Bedienmodus wechselt. Mit dem Wechsel auf den Bedienmodus wird die Bedienfunktion aktiviert, wobei gemäß der Erfindung bestimmte Funktionen der Stromquelle 1 aus Sicherheitsgründen deaktiviert werden. Beispielsweise kann der Leistungsteil der Stromquelle 1 abgestellt werden, sodass kein Schweißstrom geliefert werden kann, wenn unbeabsichtigt eine falsche Bedienung erfolgt. Auch kann beispielsweise eine Gaszufuhr unterbrochen werden.

Nach der Umschaltung auf die Bedienfunktion wird ein Referenzpunkt an der Bedien- und Anzeigeeinheit 2 - beispielsweise in der Mitte - definiert und dargestellt, von welchem ausgehend der Benutzer durch Bewegungen mit dem Schweißbrenner 4 die Bedien- und Anzeigeeinheit 2 bedienen kann. Die Bewegungen werden also relativ zum Referenzpunkt durchgeführt. Dabei entspricht der Referenzpunkt in der Bedien- und Anzeigeeinheit 2 im Wesentlichen dem Referenzpunkt im Bedienbereich. Beispielsweise wird als Referenzpunkt ein Bedienelement 3 gekennzeichnet bzw. markiert, wie durch eine farbliche Änderung oder durch eine Leuchtdiode. Somit weiß der Benutzer, an welcher Position er sich an der Bedien- und Anzeigeeinheit 2 befindet und er kann das von ihm gewünschte Bedienelement 3 durch entsprechende Bewegung des Schweißbrenners 4 markieren. Dazu ist im Schweißbrenner 4 eine Einheit 8 integriert, welche in Abhängigkeit der Position des Schweißbrenners 4 ein Signal generiert und an die Stromquelle 1 sendet, wo dieses Signal von einer Auswerteeinheit 9 ausgewertet wird. An der Bedien- und Anzeigeeinheit 2 wird das zur aktuellen Position des Schweißbrenners 4 zugeordnete Bedienelement 3 gekennzeichnet bzw. markiert. Mit dem Schweißbrenner 4 wird im Wesentlichen ein Cursor an der Bedien- und Anzeigeeinheit 2 bewegt bzw. eine Navigation durchgeführt. Die Bedien- und Anzeigeeinheit 2 kann durch ein Grafikdisplay, eine Folientastatur, einen Touchscreen oder dergl. gebildet sein.

Die Bewegungen des Schweißbrenners 4 werden bevorzugt über die Koordinaten (x, y, z) des dreidimensionalen Raums 6 und/oder über Änderungen des Winkels erfasst. Der Winkel bezieht sich dabei im Wesentlichen auf die Bewegungen des Handgelenks, wobei die Koordinaten bzw. die Position der Hand im Wesentlichen unverändert bleibt. Daher wird die Position bzw. die Koordinaten des Schweißbrenners 4 durch Änderungen des Winkels (auf/ab bzw. links/rechts) verändert, wodurch beispielsweise ein Cursor an der Bedien- und Anzeigeeinheit 2 bewegt wird. Dabei kann der Cursor kontinuierlich an der Bedien- und Anzeigeeinheit 2 bewegt oder der Cursor entsprechend der Bewegung dem nächstliegenden Bedienelement 3 der Bedien- und Anzeigeeinheit 2 zugeordnet werden. Ist das gewünschte Bedienelement 3 markiert, also die Position des Schweißbrenners 4 dem gewünschten Bedienelement 3 zugeordnet, kann der Benutzer bevorzugt durch Betätigen des Betätigungselements 10 am Schweißbrenner 4 die Zuordnung bestätigen. Das Bestätigen, beispielsweise durch einen Taster, kann unterschiedliche Vorgänge auslösen, welche vom Einsatzgebiet der Stromquelle 1 abhängen:

Ein Vorgang kann sein, dass der Parameter der Stromquelle 1 durch Betätigen eines Bedienelements 3 unmittelbar verändert wird. Hierbei entspricht das Bedienelement 3 direkt dem entsprechenden Parameter, wie dies beispielsweise bei der Auswahl des Schweißverfahrens der Fall ist.

Ein weiterer Vorgang kann sein, dass das Bedienelement 3 zwar direkt einem Parameter der Stromquelle 1 entspricht, aber zusätzlich ein Wert für den Parameter einzustellen ist. So besteht nach der Bestätigung des Bedienelements 3 an der Bedien- und Anzeigeeinheit 2 die Möglichkeit, den Wert einzustellen.

Ebenso kann auch der Vorgang ausgelöst werden, dass nach dem Bestätigen des zugeordneten Bedienelements 3 erst ein Parameter ausgewählt und anschließend durch Bewegung des Schweißbrenners 4 dessen Wert eingestellt werden muss. Dies ist auch der Fall, wenn der zugeordnete Parameter einen Menüpunkt darstellt.

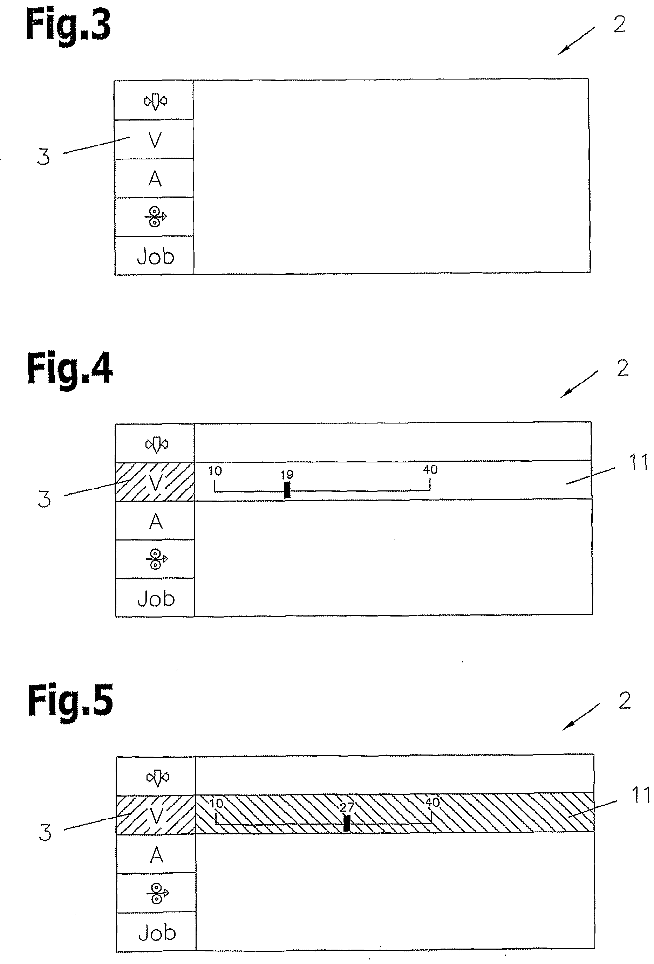

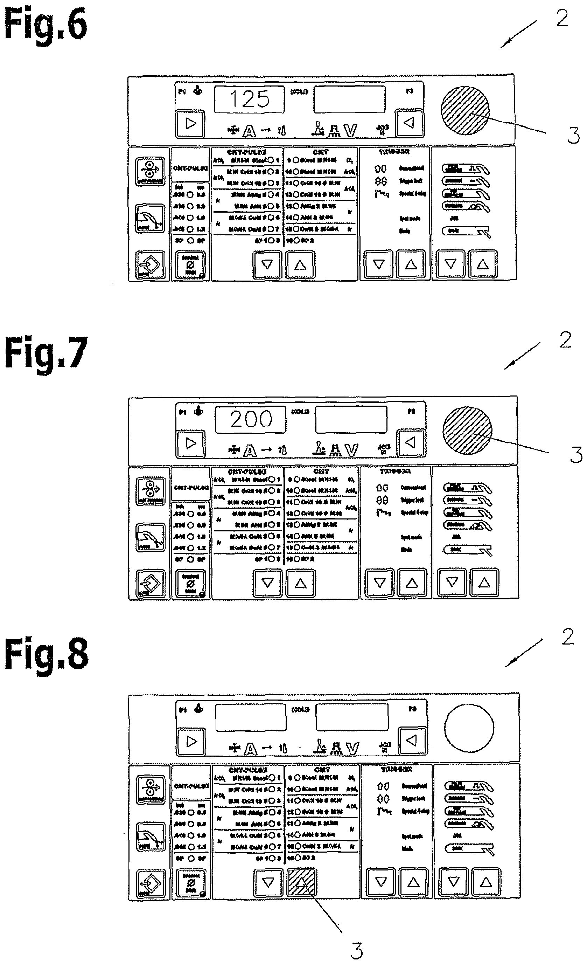

Das Einstellen des Wertes eines Parameters kann auf unterschiedliche Weise erfolgen, sodass die im Folgenden beschriebenen Einstellmöglichkeiten lediglich als Beispiele angeführt sind: So kann der Wert beispielsweise derart eingestellt werden, indem an der Bedien- und Anzeigeeinheit 2 ein Balken 11 angezeigt wird, auf welchem der aktuelle Wert markiert wird und durch Bewegungen des Schweißbrenners 4 verändert werden kann (siehe Fig. 3 bis 5). Beispielsweise wird ein Bedienelement 3 an der Bedien- und Anzeigeeinheit 2 ausgewählt. Wird die Zuordnung der Position des Schweißbrenners 4 entsprechend dem markierten Bedienelement 3 durch den Betätigungstaster 10 bestätigt, erscheint daneben ein Balken 11 mit dem aktuellen Wert. Wird nun der Balken 11 durch Bewegungen des Schweißbrenners 4 markiert und bestätigt, kann der Wert durch erneute Bewegungen verändert und bestätigt werden. Anschließend wechselt die Darstellung der Bedien- und Anzeigeeinheit 2 wieder in der Ausgangslage gemäß Fig. 3. Ebenso kann an der Bedien- und Anzeigeeinheit 2 auch ein Drehknopf als Bedienelement 3 angezeigt bzw. markiert werden, sodass durch eine entsprechende Kreisbewegung des Schweißbrenners 4 der zugehörige Wert verändert wird, wie in den Fig. 6 und 7 schematisch dargestellt. Beispielsweise kann nach Bestätigung des markierten Bedienelements 3 bzw. Drehknopfs mit einer Kreisbewegung des Schweißbrenners 4 der Wert, beispielsweise von "125" auf "200" verändert und bestätigt werden.

Ein Vorgang kann aber auch gemäß Fig. 8 sein, dass durch mehrmalige Bestätigung eines markierten Bedienelements 3 der gewünschte Parameter ausgewählt und dieser beispielsweise mit einer Leuchtdiode gekennzeichnet wird. Zum Einstellen des Wertes für den Parameter können entsprechende Elemente anstelle eines Balkens 11 angezeigt werden, mit welchen der Wert erhöht bzw. reduziert werden kann. Dies kann beispielsweise derart erfolgen, indem durch Markieren eines Elements der Wert entsprechend verändert wird. Ebenso könnte die Veränderung auch durch Betätigen des Tasters hervorgerufen werden. Der einzustellende Wert kann zusätzlich auch auf einem Display am Schweißbrenner 4 angezeigt werden. Bei diesen Einstellmöglichkeiten genügt es, lediglich zwei Dimensionen der Bewegung des Schweißbrenners 4 auszuwerten. Eine Auswertung von drei Dimensionen der Bewegung des Arbeitsgeräts 4 kann beispielsweise erforderlich sein, wenn der Arbeitspunkt für das Schweißverfahren auf einer Kennlinie verschoben wird und die Kennlinie dreidimensional auf der Bedien- und Anzeigeeinheit 2 dargestellt wird.

Der Benutzer kann also sämtliche Einstellungen an der Stromquelle 1 über die Bewegungen des Schweißbrenners 4 in seiner Hand und allenfalls einem Betätigungselement 10 am Schweißbrenner 4 vornehmen.

Dies kann beispielsweise bei einem Grafikdisplay derart erfolgen, dass ein Zeigerelement in Form eines symbolischen Schweißbrenners oder eines Pfeils über den Schweißbrenner 4 gesteuert wird, d.h., dass durch die Bewegung des Schweißbrenners 4 im Raum 6 die Position und Bewegung erfasst und auf das Zeigerelement umgesetzt wird. Bewegt man beispielsweise den Schweißbrenner 4 nach oben, so fährt das Zeigerelement an der Bedien- und Anzeigeeinheit 2 auch nach oben. Genauso kann das Zeigerelement nach links, rechts, unten im Kreis oder auf beliebige Bahnen durch eine entsprechende Bewegung des Schweißbrenners 4 in der Luft verschoben bzw. gesteuert werden. Um eine derartige Bedienung durch Bewegen des Schweißbrenners 4 im Raum 6 für den Benutzer zu erleichtern, da nahe aneinanderliegende Bedienelemente 3 über die Entfernung oft schwieriger auswählbar sind, kann beispielsweise eine besondere Bedien- und Anzeigeeinheit 2 der Stromquelle 1 verwendet werden. Hierbei kann der Benutzer beispielsweise an der Stromquelle 1 eines Schweißgeräts einstellen, dass er eine Bedienung mit dem Schweißbrenner 4 durchführen möchte, worauf eine Änderung der Bedien- und Anzeigeeinheit 2 vorgenommen wird. Beispielsweise wird beim herkömmlichen Bedienverfahren eine andere Bedien- und Anzeigeeinheit 2 dargestellt als bei einer Einstellung über den Schweißbrenner 4. Bevorzugt wird bei der Einstellung über den Schweißbrenner 4 die Bedien- und Anzeigeeinheit 2 vereinfacht ausgeführt und die auswählbaren Bedienelemente 3 darin vergrößert dargestellt. Dadurch kann der Benutzer leichter das Zeigerelement auf die entsprechenden Bedienelemente 3 bewegen. Hierzu wird die Bedien- und Anzeigeeinheit 2 auf mehreren Ebenen ausgeführt um dem Schweißer alle Einstellmöglichkeiten zur Verfügung zu stellen. Beispielsweise werden in der ersten Ebene nur die wesentlichsten Bedienelemente 3 für den Schweißstrom, die Drahtvorschubgeschwindigkeit usw., und ein weiteres Bedienelement 3 für die Umschaltung auf weitere Einstellmöglichkeiten dargestellt. Wählt der Benutzer über das Zeigerelement ein Bedienelement 3 aus und aktiviert er dieses, so öffnet sich eine neue Bedien- und Anzeigeeinheit 2, in welcher der Benutzer die Einstellung des ausgewählten Parameters über einen Balken 11, die Eingabe von Werten, über ein simuliertes Drehrad usw. durchführen kann. Damit ist auch von einer größeren Entfernung ein sicheres Ansteuern der Bedienelemente 3 möglich. Selbstverständlich kann der Schweißer auch die Größe der Symbole der Bedienelemente 3 nach seinen Anforderungen einstellen. Dementsprechend können auch wieder mehr Parameter in einer Ebene dargestellt werden. Der Schweißer kann die Bedien- und Anzeigeeinheit 2 vorzugsweise individuell an seine Erfordernisse und Fähigkeiten für die Bedienung anpassen.

Dabei sind die Bewegungen für die Bedienung beispielsweise nicht mit den Bewegungen während eines Schweißprozesses vergleichbar, sodass bereits durch diesen Unterschied eine Umschaltung auf die Funktion der Bedienung möglich wäre. Nachdem die Einstellungen vorgenommen wurden, wird erfindungsgemäss die Bedienfunktion beendet, sodass der Benutzer den Schweißbrenner 4 zweckmäßig benutzen kann, beispielsweise einen Schweißprozess durchführen kann. Das Beenden der Funktion bzw. der Wechsel in den Betriebsmodus erfolgt dabei beispielsweise durch Betätigung des Betätigungselements 10 oder durch Entfernen des Schweißbrenners 4 von der Bedien- und Anzeigeeinheit 2 umgekehrt zur Umschaltung auf die Bedienfunktion. Demnach kann mit dem Schweißbrenner 4 die Bedienung aktiviert bzw. deaktiviert oder eine Anmeldung bzw. Abmeldung für die Bedienung durchgeführt werden.

Es gibt eine Vielzahl von Möglichkeiten, wie die Einheit 8 im Schweißbrenner 4 und die Auswerteeinheit 9 in der Stromquelle 1 ausgeführt werden können und aufeinander abgestimmt sind. Beispielhaft sind im Folgenden einige angeführt:

Beispielsweise kann die Einheit 8 durch eine Infrarotkamera und die Auswerteeinheit 9 durch zwei Referenzpunkte in der Bedien- und Anzeigeeinheit 2 gebildet sein. Dadurch kann die Position - im Wesentlichen die Koordinaten im zweidimensionalen bzw. dreidimensionalen Raum - des Schweißbrenners 4 relativ zur Stromquelle 1 bestimmt werden. Dementsprechend kann die Position der Bewegung des Schweißbrenners 4 einem Bedienelement 3 zugeordnet werden. Die Übertragung der Daten erfolgt hierbei bevorzugt über einen Bus im Schlauchpaket 5.

Auch kann die Einheit 8 durch eine Infrarot-LED und die Auswerteeinheit 9 durch eine Infrarotkamera gebildet sein. Somit können wiederum die Position bzw. Positionsänderungen durch Bewegungen des Schweißbrenners 4 detektiert werden und einem Bedienelement 3 an der Bedien- und Anzeigeeinheit 2 zugeordnet werden.

Die Einheit 8 im Schweißbrenner 4 kann aber auch durch zumindest einen Sensor, wie einem Neigungssensor, einem Beschleunigungssensor oder einem Gyrosensor gebildet werden, dessen Daten durch eine entsprechende Software der Auswerteeinheit 9 verarbeitet werden. Somit kann die Position der Bewegung einem Bedienelement 3 an der Bedien- und Anzeigeeinheit 2 entsprechend zugeordnet werden.

Die Einheit 8 und die Auswerteeinheit 9 können auch generell getrennte Funktionen aufweisen. Beispielsweise kann der Abstand zwischen dem Schweißbrenner 4 und der Bedien- und Anzeigeeinheit 2 durch entsprechende Sensoren ermittelt werden und für die Ermittlung der Position des Schweißbrenners 4 eigenständige Sensoren eingesetzt werden. Auch ist es möglich, dass in der Einheit 8 Bewegungen des Schweißbrenners 4 ausgewertet und entsprechende Schritte ausgeführt werden. Die Versorgung der Einheit 8 und gegebenenfalls der Sensoren kann über das Schlauchpaket 5 erfolgen. Bevorzugt erfolgt auch die Datenübertragung über einen seriellen Datenbus im Schlauchpaket 5 oder alternativ dazu drahtlos.

Erfindungsgemäß ist auch vorgesehen, dass durch zumindest einen Ablauf einer Bewegung des Schweißbrenners 4 im Raum 6 eine Aktion bzw. ein Funktionsablauf ausgelöst wird. Dies kann beispielsweise unter den gleichen Bedingungen wie für die bereits beschriebene Bedienung der Bedien- und Anzeigeeinheit 2 erfolgen. Also zum Einen vor, nach oder zwischen einem Schweißprozess oder Ähnlichem. Zum Zweiten in einem definierten Abstand 7 zur Stromquelle 1 und zum Dritten durch Betätigen des Betätigungselements 10. Somit ist gewährleistet, dass während eines Schweißprozesses keine Bedienungs-Aktion ausgelöst wird. Eine Aktion kann durch eine abrupte Änderung der Position des Schweißbrenners 4, welche durch entsprechende Bewegungen initiiert wird, ausgelöst werden. Die abrupten Änderungen können durch Erschütterungen des Schweißbrenners 4, wie einem Klopfen, oder durch zumindest einen bestimmten Bewegungsablauf ausgelöst werden. Somit kann die Aktion beispielsweise durch eine ruckartige, seitliche oder vertikale Bewegung, eine Überschreitung eines Beschleunigungswertes oder durch eine zeitabhängige Änderung der Position ausgelöst werden. Derartige Abläufe werden vom Benutzer entsprechend zu einer Aktion konfiguriert oder es sind Abläufe bereits in einem Speicher hinterlegt. Somit kann der vom Benutzer ausgeführte Bewegungsablauf mit den hinterlegten Abläufen verglichen werden, wobei die ausgeführten Bewegungsabläufe entsprechend aufgenommen werden. Demzufolge ist kein Referenzpunkt erforderlich, da die Bedienung anhand des Bewegungsablaufs durchgeführt wird. Beispielsweise kann die Beleuchtung des Schweißbrenners 4 eingeschaltet oder eine vordefinierte Schweißeinstellung, ein sogenannter Schweißjob, verändert werden. Auch kann die Stromquelle 1 in einen "Stand-by-Betrieb" gehen, wenn der Schweißbrenner 4 eine definierte Zeit nicht bewegt wird, oder in den Betriebsmodus wechseln, wenn der Schweißbrenner 4 nach einer definierten Zeit bewegt wird. Dabei könnte auch zusätzlich ein sogenanntes Gasvorspülen gestartet werden, welches für einen Schweißprozess erforderlich ist. Es können aber auch bei abrupten Änderungen der Position des Schweißbrenners 4 Beschleunigungswerte erfasst werden und in Abhängigkeit davon bestimmte Aktionen ausgelöst werden. Ein Vergleich zwischen einer Änderung der Position des Schweißbrenners 4 und der Position der Stromquelle 1 kann beispielsweise die Aktion auslösen, dass die Stromquelle 1 abgeschaltet wird, da der Schweißbrenner 4 zu Boden gefallen ist. Demzufolge erfolgt also keine Bedienung an der Bedien- und Anzeigeeinheit 2 über den Schweißbrenner 4, sondern eine übliche Bedienung der Stromquelle 1.

Derartige Aktionen können auch im Schweißmodus bzw. Betriebsmodus erkannt werden. Ebenso können die angesprochenen Aktionen auch kombiniert werden. Beispielsweise derart, dass die Beleuchtung - wie eine Leuchtdiode (LED) - des Schweißbrenners 4 bei Bewegung des Schweißbrenners 4 eingeschaltet und entsprechend ausgeschaltet wird, wenn der Schweißbrenner 4 nicht mehr bewegt wird. Hierbei kann auch zusätzlich der "Standby-Betrieb" aktiviert werden. Weitere Aktionen können das Einfädeln des Schweißdrahtes, das Durchführen eines Gastests und/oder Ähnliches betreffen.

Selbstverständlich könnte die Einheit 8 und gegebenenfalls weitere Sensoren des Schweißbrenners 4 auch in einem Schutzhandschuh angeordnet werden, mit welchem der Benutzer den Schweißbrenner 4 hält. Dadurch kann eine bessere Schirmung erzielt und die Genauigkeit gesteigert werden. Die Versorgung kann dabei wieder über das Schlauchpaket 5 erfolgen, wobei der Schutzhandschuh mit dem Schweißbrenner 4 verbunden werden muss.

Im Allgemeinen sei noch erwähnt, dass die Bedien- und Anzeigeeinheit 2 auch von der Stromquelle 1 entfernt angeordnet sein kann, beispielsweise an einem Drahtvorschub oder einem externen Bildschirm. Weiters ist es auch möglich, dass sowohl an der Stromquelle 1 als auch am Drahtvorschubgerät eine Bedien- und Anzeigeeinheit 2 angeordnet ist. Dabei ist auch jeder Bedien- und Anzeigeeinheit 2 eine Auswerteeinheit 9 zugeordnet, wobei die Bedienung - insbesondere bei unterschiedlichen Bedien- und Anzeigeeinheiten 2 an Stromquelle 1 und Drahtvorschub - nur an einer Bedien- und Anzeigeeinheit 2 durchgeführt werden kann. Diese Entscheidung kann beispielsweise derart getroffen werden, indem die Bedien- und Anzeigeeinheit 2 jenes Geräts bedient wird, an welchem der Schweißbrenner 4 angeschlossen ist. Auch kann jene Bedien- und Anzeigeeinheit 2 bedient werden, welche den geringeren Abstand zum Schweißbrenner 4 aufweist. Dabei wird jene Auswerteeinheit 9 deaktiviert, welche jener Bedien- und Anzeigeeinheit 2 zugeordnet ist, welche nicht bedient wird. Ebenso kann eine Auswerteeinheit 9 manuell vom Benutzer deaktiviert werden. Weiters kann die Bedien- und Anzeigeeinheit 2 durch ein sogenanntes Head-up Display im Visier eines Helms des Benutzers realisiert werden. Hierbei kann die Bedienung der Stromquelle 1 direkt am Arbeitsplatz durchgeführt werden, da die Bedien- und Anzeigeeinheit 2 im Head-up Display eingeblendet wird. Die Funktion der Bedienung, wie beschrieben, bleibt dadurch im Wesentlichen unverändert. Die Bedienung kann aber auch über einen 3D-Bildschirm erfolgen, welcher die Bedien- und Anzeigeeinheit 2 entsprechend darstellt.

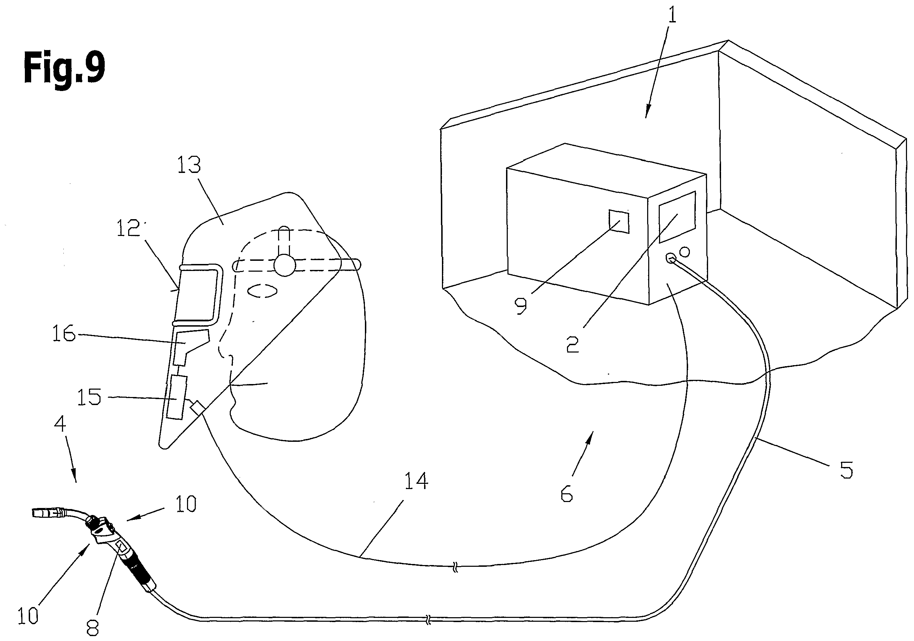

Fig. 9 zeigt den Einsatz eines sogenannten Head-up-Displays 12 eines Schweißhelms 13, Schweißschirms, Handschweißschirms oder Schutzschirms zur Ausführung eines ähnlichen Verfahrens. Zum besseren Verständnis wird das bereits Beschriebene teilweise wiederholt und es wird teilweise darauf verwiesen. Nach Beendigung des Schweißprozesses oder vor einem Schweißprozess betätigt der Schweißer bzw. Benutzer eine entsprechende Tastenkombination, insbesondere das Betätigungselement 10 am Brenner bzw. Schweißbrenner 4, sodass das Head-up-Display 12 im Visier des Schweißhelms 13 aktiviert wird und die Bedienfunktion eingeblendet wird. Vorzugsweise ist es nicht möglich, während eines Schweißprozesses durch versehentliches Drücken des entsprechenden Betätigungselements 10 das Head-up-Display 12 zu aktivieren. Dazu kann ein entsprechender Sicherheitsmodus eingebaut sein, durch welchen bei der Betätigung des Betätigungselements 10 vor der Aktivierung des Head-up-Displays 12 von der Steuervorrichtung der Stromquelle 1 überprüft wird, ob ein Lichtbogen gezündet ist oder nicht. Bei Bestehen eines Lichtbogens wird ein Einschalten des Head-up-Displays 12 unterbunden, damit das Visier dem Benutzer weiterhin als Schutz vor dem Lichtbogen dient und die Sichtverhältnisse durch den Schweißhelm 13 nicht beeinträchtigt werden und auch keine ungewollte Verstellung der Schweißparameter durch die Bewegung des Brenners während des Schweißprozesses durchgeführt werden kann.

Wie bereits oben beschrieben, wird die Bedienfunktion durch das Betätigungselement 10 am Schweißbrenner 4 aktiviert, wodurch die Stromquelle 1 in den Bedienmodus wechselt, das Head-up-Display 12 von der Stromquelle 1 aktiviert wird und zumindest ein Teil der Bedien- und Anzeigeeinheit 2 im Head-up-Display 12 angezeigt wird. Das Vorhandensein des Head-up-Displays 12 erkennt die Stromquelle 1 durch eine angeschlossene Datenverbindung und/oder eine Versorgungsverbindung. Diese Verbindungen können drahtlos und/oder leitungsgebunden - beispielsweise über einen im Schlauchpaket 5 verlaufenden Datenbus oder über eine Verbindungsleitung 14 - ausgeführt sein. Selbstverständlich ist auch eine direkte Verbindung zwischen dem Schweißbrenner 4 und dem Head-up-Display 12 möglich. Zur Einblendung der Bedienfunktion im Head-up-Display 12 ist eine entsprechende Bilderzeugungsvorrichtung 15 und gegebenenfalls eine Auswertevorrichtung 16 im Schweißhelm 13 und/oder der Stromquelle 1 angeordnet. Beispielsweise ist mit der Auswertevorrichtung 16 ein im Schweißhelm 13 integrierter Schalter verbunden, mit welchem ebenso das Head-up-Display 12 bzw. die Bedienfunktion aktiviert werden kann.

Der Schweißer hat auch die Möglichkeit am Schweißgerät bzw. der Stromquelle 1 über die Bedien- und Anzeigeeinheit 2 eine Voreinstellung für das Design der im Head-up-Display 12 eingeblendeten Bedienfunktion vorzunehmen. Dabei kann der Schweißer einstellen, ob die Oberfläche für das Head-up-Display 12 gleich aussieht wie die Ausbildung bzw. Anzeige am Schweißgerät 1, oder ob eine veränderte Darstellung eingeblendet wird. Beispielsweise kann eine vereinfachte Oberfläche mit weniger Einstellmöglichkeiten verwendet werden und bei Auswahl einer Einstellmöglichkeit ein Unterfenster geöffnet werden. Dies hat den Vorteil, dass hierzu die Symbole bzw. Bedienelemente 3 größer im Head-up-Display 12 dargestellt werden können und dem Schweißer eine leichtere Bedienung ermöglicht wird. Der Schweißer kann sich entsprechende Masken mit entsprechend ausgewählten Schweißparametern zusammenstellen, die dann im Head-up-Display 12, insbesondere als Hauptmaske, eingeblendet werden, sodass der Schweißer für seinen Schweißprozess die schnellstmöglichen Einstellungen vornehmen kann und er nicht mehrere Masken aufrufen muss. Somit ist eine beliebige Anpassung der Oberfläche der Bedienfunktion für das Head-up-Display 12 möglich.

Ebenso wird mit der Aktivierung der Bedienfunktion auch der Referenzpunkt im Head-up-Display 12 dargestellt, sodass die Navigation durchgeführt werden kann und/oder es wird eine farbliche Kennzeichnung bzw. Hinterlegung eines Bedienelements 3 vorgenommen, wobei durch Bewegung des Brenners die farbliche Kennzeichnung auf andere Bedienelemente 3 verschoben wird. Dabei können durch Bewegungen des Schweißbrenners 4 die Bedienelemente 3 im Head-up-Display 12 entsprechend deren Position zugeordnet werden und entsprechende Parameter eingestellt werden. Somit wird der Schweißbrenner 4 im Wesentlichen über dem Werkstück bzw. am Arbeitsplatz bewegt, also unabhängig von der Position der Stromquelle 1, sodass relativ zu den Bewegungen des Schweißbrenners 4 der Referenzpunkt im Head-up-Display 12 bewegt wird. Dementsprechend wird also der Referenzpunkt - wie bereits beschrieben - dem gewünschten Bedienelement 3 zugeordnet und gegebenenfalls bestätigt, sodass die Einstellungen vorgenommen werden können. Dies hat den großen Vorteil, dass der Benutzer sich nun nicht mehr vom Werkstück abwenden muss, sondern direkt über dem Werkstück durch Bewegen des Schweißbrenners in der Luft bzw. im Raum eine Bewegung des Referenzpunktes im Head-up-Display 12 bewirkt und er somit eine entsprechende Einstellung durch Auswahl eines Bedienelements 3 mit dem Referenzpunkt, bevorzugt bei Aktivierung eines Tasters, aufrufen und verändern kann. Will der Benutzer nämlich eine Einstellung nicht über das Head-up-Display 12 vornehmen bzw. ist das Head-up-Display 12 nicht aktiviert, so muss sich der Schweißer nach Beendigung des Schweißprozesses aufrichten und dem Schweißgerät, insbesondere der Bedien- und Anzeigeeinheit 2, zuwenden, sodass er den Referenzpunkt an der Bedien- und Anzeigeeinheit 2 sehen kann und durch entsprechendes Bewegen des Brenners in der Luft auf ein entsprechendes Bedienelement 3 verschieben bzw. bewegen kann. Somit dauert der Einstellvorgang wesentlich länger und der Schweißer muss auch seine Position gegenüber dem Werkstück verändern, was bei einem Einsatz eines Head-up-Displays 12 nicht notwendig ist.

Selbstverständlich können allgemein die Einstellungen, wie beispielsweise das Erhöhen oder Erniedrigen eines Wertes für den Schweißstrom, die Schweißspannung, die Drahtvorschubgeschwindigkeit oder dergleichen, auch über weitere am Schweißbrenner 4 angeordnete Standard-Schalter oder Standard-Taster - wie das zumindest eine auf der Oberseite des Schweißbrenners 4 angeordnete Betätigungselement 10 - erfolgen, wie bereits angesprochen. Der Benutzer führt die Bedienung über das Head-up-Display 12 also genauso durch, wie an der Bedien- und Anzeigeeinheit 2 der Stromquelle 1. Die Deaktivierung des Head-up-Displays 12 erfolgt im Wesentlichen wie die Deaktivierung der Bedienfunktion an der Bedien- und Anzeigeeinheit 2 der Stromquelle 1. Beispielsweise wird dazu das Betätigungselement 10 für eine definierte Zeitdauer betätigt oder ein Bedienelement 3 zum Beenden der Bedienfunktion mit dem Schweißbrenner 4 ausgewählt. Nach der Deaktivierung des Head-up-Displays 12 hat der Schweißhelm 13 wieder seine Standardfunktion, sodass der Benutzer vor einem Lichtbogen eines Schweißprozesses geschützt ist. Selbstverständlich können dennoch während des Schweißprozesses beispielsweise aktuelle oder eingestellte Werte des Schweißstroms oder Drahtvorschubgeschwindigkeit über das Head-up-Display 12 angezeigt werden, damit der Benutzer die gerade anstehenden Ist-Werte bestimmter Schweißparameter mitverfolgen kann. Bevorzugt kann dabei der Benutzer wiederum eine entsprechende Auswahl der anzuzeigenden Schweißparameter während eines Schweißprozesses auswählen. Wesentlich ist hierbei, dass diese Darstellung bevorzugt transparent und in ausgewählten Bereichen des Sichtfensters positioniert wird, sodass der Sichtbereich für den Benutzer nur unwesentlich eingeschränkt wird.

Zusammenfassend kann somit gesagt werden, dass mit dem im Schweißhelm 13 oder Schweißschirm integrierten Head-up-Display 12 der Benutzer eine uneingeschränkte Bedienung mit der kompletten Schweißausrüstung durchführen kann, ohne sich zum Schweißgerät begeben bzw. hinwenden zu müssen. Durch Betätigung eines Betätigungselements 10 wird ein Head-up-Display 12 im Schweißhelm 13 aktiviert, wobei beispielsweise ein Bedienelement 3 als Referenzpunkt bzw. mit einer farblichen Kennzeichnung für die derzeitige Position des Schweißbrenners 4 markiert wird. Durch Bewegung des Schweißbrenners 4 wird der Referenzpunkt oder die farbliche Kennzeichnung auf jenes Bedienelement 3 verschoben, an welchem die Parameter eingestellt werden sollen. Der Sicherheitsmodus bewirkt, dass eine Bedienung per Brenner während eines Schweißprozesses unterbunden wird bzw. deaktiviert ist. Selbstverständlich kann die Erfindung auch analog für einen Schneidprozess eingesetzt werden. Generell kann das Head-up-Display 12 auch durch äquivalente Anzeigeelemente, wie beispielsweise einen transparenten Bildschirm, ersetzt werden.

Selbstverständlich kann die Bedienung der Stromquelle 1 zusätzlich auch direkt über die Bedienelemente 3 an der Bedien- und Anzeigeeinheit 2 durchgeführt werden. Beispielsweise kann diese Bedienung für die Grundkonfiguration verwendet werden, während die erfindungsgemäße Bedienung mit dem Schweißbrenner 4 für Feineinstellungen nach Probeschweißungen vorgenommen wird.

Auch kann die erfindungsgemäße Bedienung derart durchgeführt werden, dass der Abstand 7 zwischen dem Schweißbrenner 4 und der Bedien- und Anzeigeeinheit 2 auf ein Minimum reduziert wird. Dabei werden die Bedienelemente 3 im Wesentlichen direkt mit dem Schweißbrenner 4 betätigt und die Einstellungen vorgenommen. Hierzu wäre auch möglich, dass zwischen der Bedien- und Anzeigeeinheit 2 und dem Schweißbrenner 4 ein Lichtbogen mit äußerst geringer Energie brennt bzw. durch Betätigen des Betätigungselements 10 ein Stromfluss zwischen dem Schweißbrenner 4 und der Bedien- und Anzeigeeinheit 2 aktiviert wird, mit welchem das gewünschte Bedienelement 3 markiert bzw. ausgewählt werden kann. Wird anschließend der Schweißbrenner 4 mit der Bedien- und Anzeigeeinheit 2 kontaktiert, erlischt der Lichtbogen aufgrund eines Kurzschlusses und das Bedienelement 3 wird betätigt.

## Patentansprüche

1. Verfahren zur Bedienung einer mit einem handbetätigten Schweißbrenner (4) verbundenen Stromquelle (1) durch einen Benutzer, wobei über Bedienelemente (3) einer Bedien- und Anzeigeeinheit (2) an der Stromquelle (1) Parameter der Stromquelle (1) eingestellt werden, und über eine in der Stromquelle (1) angeordnete Auswerteeinheit (9) eine Bewegung des Schweißbrenners (4) im Raum (6) erfasst wird, **dadurch gekennzeichnet, dass** eine Bedienfunktion an der Stromquelle (1) vor, nach oder zwischen mit dem Schweißbrenner (4) durchgeführten Arbeiten aktiviert wird und bestimmte Funktionen der Stromquelle (1) aus Sicherheitsgründen deaktiviert werden, wonach die Bewegung des Schweißbrenners (4) im Raum (6) erfasst, die aus der Bewegung des Schweißbrenners (4) resultierenden Daten in der Stromquelle (1) ausgewertet werden, und eine aus der Bewegung resultierende Position des Schweißbrenners (4) einem Bedienelement (3) der Bedien- und Anzeigeeinheit (2) zugeordnet wird, die Zuordnung der Position zu dem Bedienelement (3) an der Bedien- und Anzeigeeinheit (2) gekennzeichnet wird, und das zugeordnete Bedienelement (3) bestätigt wird, nach der Bestätigung des Bedienelements (3) der zumindest eine dem Bedienelement (3) zugeordnete Parameter der Stromquelle (1) eingestellt bzw. verändert wird, und danach die Bedienfunktion an der Stromquelle (1) wieder deaktiviert wird, und der Schweißbrenner (4) wieder zweckmäßig benutzt wird.

2. Verfahren nach Anspruch 1, **dadurch gekennzeichnet, dass** die Bedienfunktion an der Stromquelle (1) aktiviert wird, wenn ein zur Lage des Schweißbrenners (4) korrespondierender Referenzpunkt an der Bedien- und Anzeigeeinheit (2) gekennzeichnet wird.

3. Verfahren nach Anspruch 1 oder 2, **dadurch gekennzeichnet, dass** die Bedienfunktion an der Stromquelle (1) aktiviert wird, wenn ein Betätigungselement (10) am Schweißbrenner (4) betätigt wird wenn.

4. Verfahren nach einem der Ansprüche 1 bis 3, **dadurch gekennzeichnet, dass** die Bedienfunktion an der Stromquelle (1) aktiviert wird, wenn der Schweißbrenner (4) in einem definierten Abstand (7) zur Stromquelle (1) positioniert wird.

5. Verfahren nach einem der Ansprüche 1 bis 4, **dadurch gekennzeichnet, dass** die Bedienfunktion an der Stromquelle (1) aktiviert wird, indem mit dem Schweißbrenner (4) eine kurze, ruckartige Bewegung durchgeführt wird.

6. Verfahren nach einem der Ansprüche 1 bis 5, **dadurch gekennzeichnet, dass** die Bewegungen des Schweißbrenners (4) im Raum (6) dadurch erfasst werden, dass die Bewegungen zumindest in zwei Dimensionen des Raums (6) ausgewertet werden.

7. Verfahren nach einem der Ansprüche 1 bis 6, **dadurch gekennzeichnet, dass** die Bewegungen des Schweißbrenners (4) im Raum (6) dadurch erfasst werden, dass der Winkel des Schweißbrenners (4) im Raum (6) ausgewertet wird.

8. Verfahren nach einem der Ansprüche 1 bis 7, **dadurch gekennzeichnet, dass** das zugeordnete Bedienelement (3) bestätigt wird, indem ein Betätigungselement (10) am Schweißbrenner (4) betätigt wird.

9. Verfahren nach einem der Ansprüche 1 bis 8, **dadurch gekennzeichnet, dass** das zugeordnete Bedienelement (3) durch die Bewegung des Schweißbrenners (4) bestätigt wird.

10. Verfahren nach einem der Ansprüche 1 bis 9, **dadurch gekennzeichnet, dass** die aus der Bewegung des Schweißbrenners (4) resultierenden Daten über einen in einem Schlauchpaket (5) zwischen dem Schweißbrenner (4) und der Stromquelle (1) angeordneten Bus zur Stromquelle (1) übertragen werden.

11. Verfahren nach einem der Ansprüche 1 bis 10, **dadurch gekennzeichnet, dass** mit der Bedienfunktion ein mit der Stromquelle (1) verbundenes Head-up-Display (12) aktiviert wird, und zumindest ein Teil der Bedien- und Anzeigeeinheit (2) im Head-up-Display (12) angezeigt wird, und mit Deaktivierung der Bedienfunktion das Head-up-Display (12) wieder deaktiviert wird.

12. Verfahren zur Bedienung einer mit einem handbetätigten Schweißbrenner (4) verbundenen Stromquelle (1) durch einen Benutzer, wobei über Bedienelemente (3) einer Bedien- und Anzeigeeinheit (2) an der Stromquelle (1) Parameter der Stromquelle (1) eingestellt werden, und über eine in der Stromquelle (1) angeordnete Auswerteeinheit (9) eine Bewegung des Schweißbrenners (4) im Raum (6) erfasst wird, **dadurch gekennzeichnet, dass** eine Bedienfunktion an der Stromquelle (1) vor, nach oder zwischen mit dem Schweißbrenner (4) durchgeführten Arbeiten aktiviert wird und bestimmte Funktionen der Stromquelle (1) aus Sicherheitsgründen deaktiviert werden, wonach die Bewegung des Schweißbrenners (4) im Raum (6) erfasst und die erfassten Bewegungen des Schweißbrenners (4) zur Erkennung eines Bewegungsablaufs aufgenommen und mit in der Stromquelle (1) hinterlegten Bewegungsabläufen verglichen werden, und bei Übereinstimmung ein dem hinterlegten Bewegungsablauf zugeordneter Funktionsablauf ausgeführt wird, und danach die Bedienfunktion an der Stromquelle (2) wieder deaktiviert wird, und der Schweißbrenner (4) wieder zweckmäßig benutzt wird.

13. Eine Kombination eines handbetätigten Schweißbrenners und einer Stromquelle (1), welche mit dem handbetätigten Schweißbrenner (4) verbunden ist, mit einer Bedien- und Anzeigeeinheit (2) mit Bedienelementen (3) zur Einstellung von Parametern, und einer im Bereich der Bedien- und Anzeigeeinheit (2) angeordneten Auswerteeinheit (9), über welche eine Bewegung des Schweißbrenners (4) im Raum (6) erfasst wird, **dadurch gekennzeichnet, dass** eine Bedienfunktion vor, nach oder zwischen mit dem Schweißbrenner (4) durchgeführten Arbeiten aktivierbar ist und bestimmte Funktionen der Stromquelle (1) aus Sicherheitsgründen deaktivierbar sind, und dass die Auswerteeinheit (9) nach Aktivierung der Bedienfunktion zur Erfassung der Bewegung des Schweißbrenners (4) im Raum (6) und zur Auswertung der aus der Bewegung des Schweißbrenners (4) resultierenden Daten und zur Zuordnung einer aus der Bewegung resultierende Position des Schweißbrenners (4) einem Bedienelement (3) der Bedien- und Anzeigeeinheit (2), zur Kennzeichnung der Zuordnung der Position zu dem Bedienelement (3) an der Bedien- und Anzeigeeinheit (2) und zur Bestätigung des zugeordneten Bedienelements (3) und nach der Bestätigung des Bedienelements (3) zur Einstellung bzw. Veränderung des zumindest einen dem Bedienelement (3) zugeordneten Parameters ausgebildet ist, und dass danach die Bedienfunktion wieder deaktivierbar ist, und der Schweißbrenner (4) wieder zweckmäßig benutzbar ist.

## Claims

1. A method for operating a power source (1) connected to a manually actuated welding torch (4) by a user, wherein parameters of the power source (1) are set by way of operating elements (3) of an operating and display unit (2) on the power source (1) and a movement of the welding torch (4) in the space (6) is captured by an evaluation unit (9) arranged in the power source (1), **characterised in that** an operating function at the power source (1) is activated before, after or between work done with the welding torch (4) and certain functions of the power source (1) are deactivated for safety reasons, whereupon the movement of the welding torch (4) in the space (6) is captured, the data resulting from the movement of the welding torch (4) are evaluated in the power source (1) and a position of the welding torch (4) resulting from said movement is associated with an operating element (3) of the operating and display unit (2), said association of the position with the operating element (3) is denoted on the operating and display unit (2) and the associated operating element (3) is confirmed, the at least one parameter of the power source (1) associated with the operating element (3) is set and/or modified after confirming the operating element (3), and then the operating function at the power source (1) is deactivated again and the welding torch (4) is used for its original purpose again.

2. The method according to claim 1, **characterised in that** the operating function at the power source (1) is activated when a reference point corresponding to the position of the welding torch (4) is denoted on the operating and display unit (2).

3. The method according to claim 1 or 2, **characterised in that** the operating function at the power source (1) is activated when an actuation element (10) on the welding torch (4) is actuated.

4. The method according to any one of claims 1 to 3, **characterised in that** the operating function at the power source (1) is activated when the welding torch (4) is positioned at a defined distance (7) from the power source (1).

5. The method according to any one of claims 1 to 4, **characterised in that** the operating function at the power source (1) is activated by performing a short, sharp movement with the welding torch (4).

6. The method according to any one of claims 1 to 5, **characterised in that** the movements of the welding torch (4) in the space (6) are captured by said movements being evaluated in at least two dimensions of the space (6).

7. The method according to any one of claims 1 to 6, **characterised in that** the movements of the welding torch (4) in the space (6) are captured by the angle of the welding torch (4) in the space (6) being evaluated.

8. The method according to any one of claims 1 to 7, **characterised in that** the associated operating element (3) is confirmed by an actuation element (10) on the welding torch (4) being actuated.

9. The method according to any one of claims 1 to 8, **characterised in that** the associated operating element (3) is confirmed by the movement of the welding torch (4).

10. The method according to any one of claims 1 to 9, **characterised in that** the data resulting from the movement of the welding torch (4) are transferred to the power source (1) via a bus arranged in a cable-hose assembly (5) between the welding torch (4) and the power source (1).

11. The method according to any one of claims 1 to 10, **characterised in that** a head-up display (12) connected to the power source (1) is activated by way of the operating function and at least a part of the operating and display unit (2) is displayed in the head-up display (12) and the head-up display (12) is deactivated again upon deactivation of the operating function.

12. A method for operating a power source (1) connected to a manually actuated welding torch (4) by a user, wherein parameters of the power source (1) are set by way of operating elements (3) of an operating and display unit (2) on the power source (1) and a movement of the welding torch (4) in the space (6) is captured by an evaluation unit (9) arranged in the power source (1), **characterised in that** an operating function at the power source (1) is activated before, after or between work done with the welding torch (4) and certain functions of the power source (1) are deactivated for safety reasons, whereupon the movement of the welding torch (4) in the space (6) is captured and the captured movements of the welding torch (4) are recorded and compared to motion events stored in the power source (1) in order to identify a motion event, with a functional event associated with the stored motion event being carried out if matches are detected, and then the operating function at the power source (2) is deactivated again and the welding torch (4) is used for its original purpose again.

13. A combination of a manually actuated welding torch and a power source (1) connected to the manually actuated welding torch (4), having an operating and display unit (2) with operating elements (3) for setting parameters and an evaluation unit (9) arranged in the region of the operating and display unit (2) by which a movement of the welding torch (4) in the space (6) is captured, **characterised in that** an operating function may be activated before, after or between work done with the welding torch (4) and certain functions of the power source (1) may be deactivated for safety reasons, and that upon activation of the operating function the evaluation unit (9) is designed for capturing the movement of the welding torch (4) in the space (6) and for evaluating the data resulting from the movement of the welding torch (4) and for associating a position of the welding torch (4) resulting from the movement with an operating element (3) of the operating and display unit (2), for denoting said association of the position with the operating element (3) on the operating and display unit (2) and for confirming the associated operating element (3) and, after confirming the operating element (3), for setting and/or modifying the at least one parameter associated with the operating element (3), and that afterwards the operating function may be deactivated again and the welding torch (4) may be used for its original purpose again.

## Revendications

1. Procédé de commande d'une source de courant (1) connectée avec un chalumeau de soudure à commande manuelle (4) par un utilisateur, des éléments de commande (3) d'une unité de commande et d'affichage (2) sur la source de courant (1) permettant de régler des paramètres de la source de courant (1), et une unité d'analyse (9), disposée dans la source de courant (1), permettant de mesurer un mouvement du chalumeau de soudure (4) dans l'espace (6), **caractérisé en ce qu'**une fonction de commande sur la source de courant (1) est activée avant, après ou entre les travaux effectués avec le chalumeau de soudure (4) et certaines fonctions de la source de courant (1) sont désactivées pour des raisons de sécurité, le mouvement du chalumeau de soudure (4) dans l'espace (6) est ensuite mesuré, les données résultant du mouvement du chalumeau de soudure (4) sont analysées dans la source de courant (1) et une position résultant du mouvement du chalumeau de soudure (4) est attribuée à un élément de commande (3) de l'unité de commande et d'affichage (2), l'attribution de la position à l'élément de commande (3) est identifiée sur l'unité de commande et d'affichage (2) et l'élément de commande (3) attribué est confirmé, après la confirmation de l'élément de commande (3), l'au moins un paramètre, attribué à l'élément de commande (3), de la source de courant (1) est réglé ou modifié, puis la fonction de commande est à nouveau désactivée sur la source de courant (1) et le chalumeau de soudure (4) est à nouveau utilisé de manière appropriée.

2. Procédé selon la revendication 1, **caractérisé en ce que** la fonction de commande sur la source de courant (1) est activée lorsqu'un point de référence correspondant à la position du chalumeau de soudure (4) est identifié sur l'unité de commande et d'affichage (2).

3. Procédé selon la revendication 1 ou 2, **caractérisé en ce que** la fonction de commande sur la source de courant (1) est activée lorsqu'un élément d'actionnement (10) est actionné sur le chalumeau de soudure (4).

4. Procédé selon l'une des revendications 1 à 3, **caractérisé en ce que** la fonction de commande sur la source de courant (1) est activée lorsque le chalumeau de soudure (4) est positionné à une distance définie (7) par rapport à la source de courant (1).

5. Procédé selon l'une des revendications 1 à 4, **caractérisé en ce que** la fonction de commande sur la source de courant (1) est activée en effectuant, avec le chalumeau de soudure (4), un mouvement bref et brusque.

6. Procédé selon l'une des revendications 1 à 5, **caractérisé en ce que** les mouvements du chalumeau de soudure (4) dans l'espace (6) sont mesurés grâce au fait que les mouvements sont analysés dans au moins deux dimensions de l'espace (6).

7. Procédé selon l'une des revendications 1 à 6, **caractérisé en ce que** les mouvements du chalumeau de soudure (4) dans l'espace (6) sont mesurés grâce au fait que l'angle du chalumeau de soudure (4) dans l'espace (6) est analysé.

8. Procédé selon l'une des revendications 1 à 7, **caractérisé en ce que** l'élément de commande attribué (3) est confirmé en actionnant un élément d'actionnement (10) sur le chalumeau de soudure (4).

9. Procédé selon l'une des revendications 1 à 8, **caractérisé en ce que** l'élément de commande attribué (3) est confirmé par les mouvements du chalumeau de soudure (4).

10. Procédé selon l'une des revendications 1 à 9, **caractérisé en ce que** les données résultant du mouvement du chalumeau de soudure (4) sont transmises, par l'intermédiaire d'un bus disposé dans un faisceau (5) entre le chalumeau de soudure (4) et la source de courant (1), à la source de courant (1).

11. Procédé selon l'une des revendications 1 à 10, **caractérisé en ce que**, avec la fonction de commande, un affichage tête haute (12) connecté à la source de courant (1) est activé, et au moins une partie de l'unité de commande et d'affichage (2) est affichée dans l'affichage tête haute (12) et, lors de la désactivation de la fonction de commande, l'affichage tête haute (12) est également désactivé.

12. Procédé de commande d'une source de courant (1) connectée avec un chalumeau de soudure à commande manuelle (4) par un utilisateur, des éléments de commande (3) d'une unité de commande et d'affichage (2) au niveau de la source de courant (1) permettant de régler des paramètres de la source de courant (1), et une unité d'analyse (9), disposée dans la source de courant (1), permettant de mesurer un mouvement du chalumeau de soudure (4) dans l'espace (6), **caractérisé en ce qu'**une fonction de commande sur la source de courant (1) est activée avant, après ou entre des travaux effectués avec le chalumeau de soudure (4) et certaines fonctions de la source de courant (1) sont désactivées pour des raisons de sécurité, le mouvement du chalumeau de soudure (4) dans l'espace (6) est ensuite mesuré et les mouvements mesurés du chalumeau de soudure (4) sont enregistrés pour la détection d'une trajectoire de mouvement et comparés avec des trajectoires de mouvements enregistrés dans la source de courant (1) et, dans le cas d'une correspondance, un processus de fonctionnement correspondant à la trajectoire de mouvement enregistrée est exécuté, puis la fonction de commande sur la source de courant (2) est à nouveau désactivée et le chalumeau de soudure (4) est à nouveau utilisé de manière approprié.

13. Combinaison d'un chalumeau de soudure à commande manuelle et d'une source de courant (1), qui est connectée avec le chalumeau de soudure à commande manuelle (4), avec une unité de commande et d'affichage (2) avec des éléments de commande (3) pour le réglage de paramètres et une unité d'analyse (9), disposée au niveau de l'unité de commande et d'affichage (2), par l'intermédiaire de laquelle un mouvement du chalumeau de soudure (4) dans l'espace (6) est mesuré, **caractérisée en ce qu'**une fonction de commande peut être activée avant, après ou entre des travaux effectués avec le chalumeau de soudure (4) et certaines fonctions de la source de courant (1) peuvent être désactivées pour des raisons de sécurité et **en ce que** l'unité d'analyse (9), après l'activation de la fonction de commande, est conçue pour la mesure du mouvement du chalumeau de soudure (4) dans l'espace (6) et pour l'analyse des données résultant du mouvement du chalumeau de soudure (4) et pour l'attribution d'une position, résultant du mouvement du chalumeau de soudure (4), à un élément de commande (3) de l'unité de commande et d'affichage (2), pour l'identification de l'attribution de la position par rapport à l'élément de commande (3) sur l'unité de commande et d'affichage (2) et pour la confirmation de l'élément de commande attribué (3) et après la confirmation de l'élément de commande (3) pour le réglage ou la modification de l'au moins un paramètre attribué à l'élément de commande (3) et **en ce que** la fonction de commande peut ensuite être à nouveau désactivée et le chalumeau de soudure (4) peut à nouveau être utilisé de manière appropriée.
